# EUROPEAN PATENT APPLICATION

(11) **EP 4 831 150 A1**
(43) Date of publication of application: **28.10.2026**
(21) Application number: 23962035.4
(22) Date of filing: 23.12.2023
(51) Int. Cl.: C07C 1/20, C07C 4/06, B01J 8/24, C07C 11/04, C07C 11/06

(54) **METHOD FOR PREPARING LOW-CARBON OLEFINS BY COUPLING METHANOL WITH MIXED HYDROCARBONS**

(30) Priority: 18.12.2023 CN 202311746714
(71) Applicant: Dalian Institute Of Chemical Physics, Chinese Academy Of Sciences, Liaoning 116023 (CN)
(72) Inventor: ZHANG, Tao, Dalian, Liaoning 116023 (CN); YE, Mao, Dalian, Liaoning 116023 (CN); ZHANG, Jinling, Dalian, Liaoning 116023 (CN); MA, Xiangang, Dalian, Liaoning 116023 (CN); WANG, Xiangao, Dalian, Liaoning 116023 (CN); TANG, Hailong, Dalian, Liaoning 116023 (CN); XU, Shuliang, Dalian, Liaoning 116023 (CN); WANG, Jing, Dalian, Liaoning 116023 (CN); ZHANG, Cheng, Dalian, Liaoning 116023 (CN); JIA, Jinming, Dalian, Liaoning 116023 (CN); LIU, Zhongmin, Dalian, Liaoning 116023 (CN)
(74) Representative: Barker Brettell LLP
(86) International application number: PCT/CN2023/141302
(87) International publication number: WO 2025/129713

(57) **Abstract**

Disclosed is a method for preparing low-carbon olefins by coupling methanol with mixed hydrocarbons. The method comprises: introducing gasified methanol and mixed hydrocarbon raw materials into a reaction zone for contact with a catalyst for a reaction to generate stream I containing a product gas and the catalyst; subjecting stream I to gas-solid separation, then the catalyst entering a catalyst residence zone, and the product gas entering a downstream section; and part of the catalyst in the catalyst residence zone entering the reaction zone, conveying part of the catalyst to a fluidized bed regenerator for regeneration, and the regenerated catalyst entering a fluidized bed reactor. By means of a fluidized bed reaction-regeneration process, the method achieves continuous preparation of low-carbon olefins by coupling methanol with mixed hydrocarbons. The reaction of preparing the low-carbon olefins from methanol is exothermic, thus providing heat for the catalytic cracking of the mixed hydrocarbons to prepare the low-carbon olefins, achieving self-heat balance. The reaction contact time of the product gas in the reactor is less than 2 s, the carbon-based selectivity for "ethylene + propylene" is greater than or equal to 75% wt, the single-pass conversion rate of methanol is greater than or equal to 99% wt, and the single-pass conversion rate of the mixed hydrocarbons is greater than or equal to 50% wt.

## Description

### TECHNICAL FIELD

The present application relates to the field of chemical catalysis technology, and in particular, to a method for preparing low-carbon olefins by coupling methanol with mixed hydrocarbons.

### BACKGROUND

Olefins are important basic organic chemical raw materials and the cornerstone of the modern chemical industry. Olefin production technology routes mainly include naphtha cracking to produce olefins, methanol to olefins, propane dehydrogenation to produce propylene, alkane cracking to produce olefins, and olefin cracking to produce olefins. Low-carbon olefins are very active and prone to polymerization, alkylation, aromatization and other reactions, generating by-products and reducing the yield of low-carbon olefins.

Methanol to olefins (MTO) technology targets ethylene and propylene as products. Under the action of molecular sieve catalysts, methanol is converted into a mixture containing ethylene, propylene, butene, pentene, alkanes and other components. The methanol to olefins reaction is a strongly exothermic reaction, and it is usually necessary to remove the heat released by the reaction and coke combustion through a heat exchanger.

UOP has developed olefin catalytic cracking (OCP) technology, in which C₄-C₈ olefin feedstocks are converted to ethylene and propylene under high temperature and low pressure conditions, with a propylene to ethylene ratio of about 4:1. The OCP unit has two radial flow reactors, one of which is in operation online and the other is regenerated and on standby. The regeneration cycle of the reactor is 48 hours. The switching between online operation and offline regeneration of the reactor is achieved through valve groups installed at the inlet and outlet of the reactor.

Sinopec has developed olefin catalytic cracking (OCC) technology, in which olefins contained in mixed C₄-C₆ feedstocks are cracked into propylene, ethylene, etc. under the action of molecular sieve catalysts. The OCC unit has two fixed bed reactors, one in operation and one on standby. The reaction system operates in a continuous switching mode, and the catalyst regeneration process is controlled by an automatic switching regeneration program.

The catalytic cracking reaction to produce low-carbon olefins is a strongly endothermic process. Radial flow reactors and fixed bed reactors generally achieve heat supply by increasing the feed temperature of the feedstock. However, if the preheating temperature of feedstock components such as olefins, diolefins, and alkynes is too high, coking will occur, affecting the continuous operation of the unit. In addition, the catalyst gradually loses activity during the reaction process, and continuous operation needs to be achieved by switching reactors. The problems of heat supply and deactivation limit the development and progress of radial flow reactor technology and fixed bed reactor technology.

### SUMMARY

In view of this, the present application provides a method for preparing low-carbon olefins by coupling methanol with mixed hydrocarbons, with the main purpose of solving the technical problems of low yield of low-carbon olefins, low conversion rate of mixed hydrocarbons, and heat supply for mixed hydrocarbon cracking.

Low-carbon olefins are very active and prone to polymerization, alkylation, aromatization and other reactions, generating by-products and reducing the yield of low-carbon olefins. Reaction contact time is an important factor affecting the selectivity of low-carbon olefins in the catalytic cracking process of mixed hydrocarbons to prepare low-carbon olefins. Increasing the superficial gas velocity in the reaction zone of the fluidized bed reactor can reduce the reaction contact time and improve the selectivity of low-carbon olefins. However, the bed density of the reaction zone usually decreases significantly at the same time, and the catalyst inventory also decreases significantly, which in turn leads to a decrease in the conversion rate of the cracking feedstock. That is, there is a negative correlation between the selectivity of low-carbon olefins and the feedstock conversion rate.

In order to improve the yield of low-carbon olefins and the conversion rate of feedstock, the present application provides a high-density fast fluidized bed reactor. The high-density fast fluidized bed reactor comprises a reactor outer shell, a reactor inner shell, and a transport pipe.

The reactor outer shell is provided with at least a feedstock inlet, a catalyst inlet, a gas phase product outlet, and a catalyst outlet. The reactor outer shell encloses an internal region. The reactor inner shell is located in the lower part of the internal region, and the region enclosed by the reactor inner shell is a reaction zone. The transport pipe is located in the middle and upper part of the internal region, and the bottom of the transport pipe is in communication with the reaction zone. The region enclosed by the reactor outer shell and the transport pipe is a gas-solid separation zone. The transport pipe is provided with an outlet, and the transport pipe is in communication with the gas-solid separation zone. The annular region enclosed by the reactor outer shell and the reactor inner shell is a catalyst residence zone. The bottom of the reaction zone and the bottom of the catalyst residence zone are in communication. The catalyst residence zone and the gas-solid separation zone are in communication and located below the gas-solid separation zone.

Optionally, the high-density fast fluidized bed reactor comprises a catalyst distribution pipe, a fluidizing steam distributor, and a feedstock distributor. The catalyst distribution pipe passes through the reactor inner shell to connect the catalyst residence zone and the reaction zone. The fluidizing steam distributor is disposed at the bottom of the catalyst residence zone. The feedstock distributor is disposed at the bottom of the reaction zone.

Optionally, the lower surface of the catalyst distribution pipe is provided with through holes.

Optionally, the high-density fast fluidized bed reactor comprises a first gas-solid separation device and a second gas-solid separation device. The first gas-solid separation device and the second gas-solid separation device are located in the gas-solid separation zone. The inlet of the first gas-solid separation device is in communication with the outlet of the transport pipe. The catalyst outlet of the first gas-solid separation device is located in the lower part of the gas-solid separation zone, and the gas outlet of the first gas-solid separation device is located in the upper part of the gas-solid separation zone.

Preferably, the inlet of the second gas-solid separation device is located in the upper part of the gas-solid separation zone. The catalyst outlet of the second gas-solid separation device is located in the lower part of the gas-solid separation zone.

Optionally, the first gas-solid separation device is an inertial separator.

Optionally, the second gas-solid separation device is one or more groups of gas-solid cyclone separators, each group of gas-solid cyclone separators comprising a first-stage gas-solid cyclone separator and a second-stage gas-solid cyclone separator.

Optionally, the high-density fast fluidized bed reactor comprises a reactor gas collection chamber and a product gas transport pipe. The reactor gas collection chamber is located at the top of the high-density fast fluidized bed reactor. The product gas transport pipe is connected to the top of the reactor gas collection chamber. The gas outlet of the second gas-solid separation device is in communication with the reactor gas collection chamber.

In a second aspect, the present application provides an apparatus for preparing low-carbon olefins from methanol and mixed hydrocarbons. The apparatus comprises the above-mentioned high-density fast fluidized bed reactor and a fluidized bed regenerator.

A catalyst extraction pipe passes through the reactor outer shell and is located in the lower part of the catalyst residence zone. The catalyst extraction pipe is in communication with a reactor stripper. The inlet of a spent slide valve is connected to the bottom of the reactor stripper. The outlet of the spent slide valve is connected to the inlet of a spent agent transport pipe. The outlet of the spent agent transport pipe is in communication with the fluidized bed regenerator.

A regenerator stripper is located at the bottom of the fluidized bed regenerator. The inlet of a regenerated slide valve is connected to the bottom of the regenerator stripper. The outlet of the regenerated slide valve is connected to the inlet of a regenerated agent transport pipe. The outlet of the regenerated agent transport pipe is connected to the lower part of the gas-solid separation zone of the high-density fast fluidized bed reactor.

Optionally, the fluidized bed regenerator comprises a regenerator shell, a regenerator distributor, a third gas-solid separation device, a regenerator gas collection chamber, and a flue gas transport pipe. The regenerator distributor is located at the bottom of the fluidized bed regenerator. The third gas-solid separation device is located in the upper part of the fluidized bed regenerator. The inlet of the third gas-solid separation device is located in the upper part of the fluidized bed regenerator. The gas outlet of the third gas-solid separation device is in communication with the regenerator gas collection chamber. The catalyst outlet of the third gas-solid separation device is located in the lower part of the fluidized bed regenerator. The regenerator gas collection chamber is located at the top of the fluidized bed regenerator. The flue gas transport pipe is connected to the top of the regenerator gas collection chamber.

Optionally, the inlet pipe of the regenerator stripper penetrates the regenerator shell and opens above the regenerator distributor.

Optionally, the third gas-solid separation device employs one or more groups of gas-solid cyclone separators, each group of gas-solid cyclone separators comprising a first-stage gas-solid cyclone separator and a second-stage gas-solid cyclone separator.

In a third aspect, the present application provides a method for preparing low-carbon olefins by coupling methanol with mixed hydrocarbons, which is carried out using the above-mentioned apparatus.

Optionally, the method for preparing low-carbon olefins comprises the following steps: introducing gasified feedstock comprising methanol and mixed hydrocarbons into the reaction zone, contacting with the catalyst, reacting to generate stream I containing product gas of low-carbon olefins and the catalyst; after gas-solid separation of stream I, obtaining the product gas and the catalyst; transporting the product gas to a downstream section; and part of the catalyst entering the reaction zone, another part of the catalyst undergoing regeneration, and the regenerated catalyst entering the reaction zone.

Optionally, stream I enters the first gas-solid separation device through the transport pipe, and the catalyst after the first gas-solid separation enters the catalyst residence zone.

Optionally, the mixed hydrocarbons in the feedstock are C₄+ hydrocarbons, comprising alkanes and olefins with carbon atom numbers ≥ 4.

Optionally, the catalyst is selected from ZSM-5 molecular sieve catalyst.

Optionally, the process operating conditions of the reaction zone of the high-density fast fluidized bed reactor include: superficial gas velocity of 1.5-7.0 m/s, temperature of 500-680°C, pressure of 50-250 kPa, bed density of 150-500 kg/m³, and reaction contact time of 0.5-2 s.

Optionally, the superficial gas velocity of the reaction zone is selected from any value among 1.5 m/s, 2 m/s, 2.5 m/s, 3 m/s, 3.5 m/s, 4 m/s, 4.5 m/s, 5 m/s, 5.5 m/s, 6 m/s, 6.5 m/s, 7 m/s, or a range value between any two thereof.

Optionally, the temperature of the reaction zone is selected from any value among 500°C, 550°C, 600°C, 650°C, 680°C, or a range value between any two thereof.

Optionally, the pressure of the reaction zone is selected from any value among 50 kPa, 100 kPa, 150 kPa, 200 kPa, 250 kPa, or a range value between any two thereof.

Optionally, the bed density of the reaction zone is selected from any value among 150 kg/m³, 200 kg/m³, 250 kg/m³, 300 kg/m³, 350 kg/m³, 400 kg/m³, 450 kg/m³, 500 kg/m³, or a range value between any two thereof.

Optionally, the reaction contact time of the reaction zone is selected from any value among 0.5 s, 1 s, 1.5 s, 2 s, or a range value between any two thereof.

Optionally, the process operating conditions of the catalyst residence zone include: superficial gas velocity of 0.02-0.2 m/s, temperature of 500-680°C, and bed density of 600-800 kg/m³.

Optionally, the catalyst circulation intensity flowing from the catalyst residence zone to the reaction zone is 500-1000 kg/(m²·s).

Optionally, the superficial gas velocity of the catalyst residence zone is selected from any value among 0.02 m/s, 0.05 m/s, 0.08 m/s, 0.1 m/s, 0.12 m/s, 0.15 m/s, 0.18 m/s, 0.20 m/s, or a range value between any two thereof.

Optionally, the temperature of the catalyst residence zone is selected from any value among 500°C, 550°C, 600°C, 650°C, 680°C, or a range value between any two thereof.

Optionally, the bed density of the catalyst residence zone is selected from any value among 600 kg/m³, 650 kg/m³, 700 kg/m³, 750 kg/m³, 800 kg/m³, or a range value between any two thereof.

Optionally, the catalyst circulation intensity is selected from any value among 500, 550, 600, 650, 700, 750, 800, 850, 900, 950, 1000, or a range value between any two thereof, in units of kg/(m²·s).

Optionally, the process operating conditions of the fluidized bed regenerator are: superficial gas velocity of 0.5-2.0 m/s, regeneration temperature of 600-750°C, regeneration pressure of 50-250 kPa, and bed density of 200-800 kg/m³.

Optionally, the superficial gas velocity of the fluidized bed regenerator is selected from any value among 0.5 m/s, 1 m/s, 1.5 m/s, 2 m/s, or a range value between any two thereof.

Optionally, the regeneration temperature of the fluidized bed regenerator is selected from any value among 600°C, 650°C, 700°C, 750°C, or a range value between any two thereof.

Optionally, the regeneration pressure of the fluidized bed regenerator is selected from any value among 50 kPa, 100 kPa, 150 kPa, 200 kPa, 250 kPa, or a range value between any two thereof.

Optionally, the bed density of the fluidized bed regenerator is selected from any value among 200kg/m³, 250kg/m³, 300 kg/m³, 350 kg/m³, 400 kg/m³, 450 kg/m³, 500 kg/m³, 550 kg/m³, 600 kg/m³, 650 kg/m³, 700 kg/m³, 750 kg/m³, 800 kg/m³ or a range value between any two thereof.

Optionally, the regeneration gas is air.

Optionally, steam enters the catalyst residence zone from the fluidizing steam distributor, and the steam carries part of the catalyst in the catalyst residence zone into the gas-solid separation zone; the product gas and the steam carrying part of the catalyst in the gas-solid separation zone enter the second gas-solid separation device, and after the second gas-solid separation, the gas enters the reactor gas collection chamber, and the separated catalyst returns to the catalyst residence zone.

Optionally, the product gas and steam after the second gas-solid separation enter the downstream section through the product gas transport pipe; a first part of the catalyst in the catalyst residence zone enters the reaction zone through the catalyst distribution pipe; a second part of the catalyst in the catalyst residence zone enters the bottom of the reaction zone through the bottom of the catalyst residence zone; and a third part of the catalyst in the catalyst residence zone enters the reactor stripper through the catalyst extraction pipe.

The present application provides a specific apparatus for preparing low-carbon olefins from methanol and mixed hydrocarbons, comprising: a reactor outer shell, a reactor inner shell, a transport pipe, a feedstock distributor, a first gas-solid separation device, a fluidizing steam distributor, a catalyst distribution pipe, a second gas-solid separation device, a reactor gas collection chamber, a product gas transport pipe, a catalyst extraction pipe, a reactor stripper, a spent slide valve, and a spent agent transport pipe.

The region enclosed by the reactor inner shell is the reaction zone. The annular region enclosed by the reactor outer shell and the reactor inner shell is the catalyst residence zone. The bottom of the reaction zone and the bottom of the catalyst residence zone are in communication. The region enclosed by the reactor outer shell and the transport pipe is the gas-solid separation zone. The catalyst residence zone and the gas-solid separation zone are in communication and located below the gas-solid separation zone.

The feedstock distributor is located at the bottom of the reaction zone. The transport pipe is located in the central region of the middle and upper part of the high-density fast fluidized bed reactor. The bottom end of the transport pipe is connected to the top of the reaction zone. The outlet of the transport pipe is connected to the inlet of the first gas-solid separation device. The first gas-solid separation device is located in the gas-solid separation zone. The catalyst outlet of the first gas-solid separation device is located in the lower part of the gas-solid separation zone. The gas outlet of the first gas-solid separation device is located in the upper part of the gas-solid separation zone.

The fluidizing steam distributor is located at the bottom of the catalyst residence zone. The catalyst distribution pipe passes through the reactor inner shell to connect the catalyst residence zone and the reaction zone. The lower surface of the catalyst distribution pipe is provided with openings.

The second gas-solid separation device is located in the gas-solid separation zone. The inlet of the second gas-solid separation device is located in the gas-solid separation zone. The gas outlet of the second gas-solid separation device is connected to the reactor gas collection chamber. The catalyst outlet of the second gas-solid separation device is located in the lower part of the gas-solid separation zone. The reactor gas collection chamber is located at the top of the high-density fast fluidized bed reactor. The product gas transport pipe is connected to the top of the reactor gas collection chamber.

The catalyst extraction pipe passes through the reactor outer shell and is located in the lower part of the catalyst residence zone. The reactor stripper is connected to the catalyst extraction pipe. The inlet of the spent slide valve is connected to the bottom of the reactor stripper. The outlet of the spent slide valve is connected to the inlet of the spent agent transport pipe. The outlet of the spent agent transport pipe is connected to the fluidized bed regenerator.

The first gas-solid separation device employs an inertial separator to achieve rapid separation of product gas and catalyst. The second gas-solid separation device employs one or more groups of gas-solid cyclone separators, each group of gas-solid cyclone separators comprising a first-stage gas-solid cyclone separator and a second-stage gas-solid cyclone separator.

The fluidized bed regenerator for regenerating the catalyst comprises: a regenerator shell, a regenerator distributor, a third gas-solid separation device, a regenerator gas collection chamber, a flue gas transport pipe, a regenerator stripper, a regenerated slide valve, and a regenerated agent transport pipe.

The regenerator distributor is located at the bottom of the fluidized bed regenerator. The third gas-solid separation device is located in the upper part of the fluidized bed regenerator. The inlet of the third gas-solid separation device is located in the upper part of the fluidized bed regenerator. The gas outlet of the third gas-solid separation device is connected to the regenerator gas collection chamber. The catalyst outlet of the third gas-solid separation device is located in the lower part of the fluidized bed regenerator. The regenerator gas collection chamber is located at the top of the fluidized bed regenerator. The flue gas transport pipe is connected to the top of the regenerator gas collection chamber.

The regenerator stripper is located outside the regenerator shell. The inlet pipe of the regenerator stripper penetrates the regenerator shell and opens above the regenerator distributor. The inlet of the regenerated slide valve is connected to the bottom of the regenerator stripper. The outlet of the regenerated slide valve is connected to the inlet of the regenerated agent transport pipe. The outlet of the regenerated agent transport pipe is connected to the lower part of the gas-solid separation zone of the high-density fast fluidized bed reactor.

The third gas-solid separation device employs one or more groups of gas-solid cyclone separators, each group of gas-solid cyclone separators comprising a first-stage gas-solid cyclone separator and a second-stage gas-solid cyclone separator.

The present application provides a specific method for preparing low-carbon olefins by coupling methanol with mixed hydrocarbons, comprising:
a. The catalyst from the regenerated agent transport pipe enters the gas-solid separation zone of the high-density fast fluidized bed reactor, and then enters the catalyst residence zone. The gasified feedstock enters the reaction zone from the feedstock distributor, contacts with the catalyst, and generates product gas containing low-carbon olefins. The product gas carries the catalyst through the transport pipe into the first gas-solid separation device. After gas-solid separation, the catalyst enters the catalyst residence zone. Steam enters the catalyst residence zone from the fluidizing steam distributor, and the steam carries a small amount of catalyst from the catalyst residence zone into the gas-solid separation zone. The product gas and steam carrying the catalyst in the gas-solid separation zone enter the second gas-solid separation device. After gas-solid separation, the gas enters the reactor gas collection chamber, and the catalyst returns to the catalyst residence zone. The product gas and steam enter the downstream section through the product gas transport pipe. The catalyst in the catalyst residence zone enters the reaction zone through the catalyst distribution pipe. The catalyst in the catalyst residence zone enters the bottom of the reaction zone through the bottom of the catalyst residence zone. The catalyst in the catalyst residence zone enters the reactor stripper through the catalyst extraction pipe. After stripping, the catalyst then enters the middle part of the fluidized bed regenerator through the spent slide valve and the spent agent transport pipe.
b. The regeneration gas enters the bottom of the fluidized bed regenerator from the regenerator distributor. In the fluidized bed regenerator, the regeneration gas contacts with the catalyst, and the carbon deposits on the catalyst are burned and eliminated. The flue gas formed by combustion carries the catalyst into the third gas-solid separation device. After gas-solid separation, the flue gas enters the regenerator gas collection chamber and then enters the downstream flue gas treatment system through the flue gas transport pipe. The catalyst returns to the bottom of the fluidized bed regenerator. The catalyst in the fluidized bed regenerator enters the regenerator stripper. After stripping, it then enters the high-density fast fluidized bed reactor through the regenerated slide valve and the regenerated agent transport pipe.

The feedstock is methanol and mixed hydrocarbons. Preferably, the mixed hydrocarbons are C₄+ hydrocarbons, comprising alkanes and olefins with carbon atom numbers ≥ 4. The catalyst is a ZSM-5 molecular sieve catalyst.

The process operating conditions of the reaction zone of the high-density fast fluidized bed reactor are: superficial gas velocity of 1.5-7.0 m/s, temperature of 500-680°C, pressure of 50-250 kPa, bed density of 150-500 kg/m³, and reaction contact time of 0.5-2 s.

The process operating conditions of the catalyst residence zone are: superficial gas velocity of 0.02-0.2 m/s, temperature of 500-680°C, and bed density of 600-800 kg/m ³. The catalyst circulation intensity flowing from the catalyst residence zone to the reaction zone is 500-1000 kg/(m²·s). The regeneration gas is air.

The process operating conditions of the fluidized bed regenerator are: superficial gas velocity of 0.5-2.0 m/s, regeneration temperature of 600-750°C, regeneration pressure of 50-250 kPa, and bed density of 200-800 kg/m³.

In the method of the present application, the single-pass conversion rate of methanol is ≥ 99% wt, the single-pass conversion rate of mixed hydrocarbons is ≥ 50% wt, the carbon-based selectivity of "ethylene + propylene" is ≥ 75% wt, and the propylene/ethylene (mass ratio) is ≥ 3.

In the method of the present application, the calculation methods for the single-pass conversion rate of methanol, the single-pass conversion rate of mixed hydrocarbons, and the carbon-based selectivity of "ethylene + propylene" are as follows: 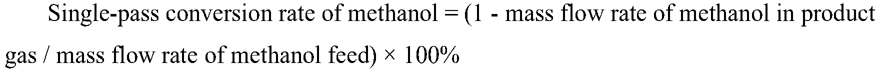 Single-pass conversion rate of methanol = (1 - mass flow rate of methanol in product gas / mass flow rate of methanol feed) × 100% 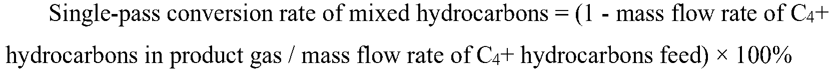 Single-pass conversion rate of mixed hydrocarbons = (1 - mass flow rate of C4+ hydrocarbons in product gas / mass flow rate of C4+ hydrocarbons feed) × 100% 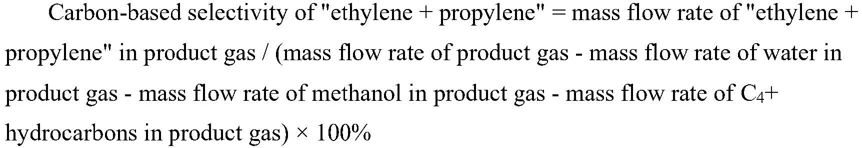 Carbon-based selectivity of "ethylene + propylene" = mass flow rate of "ethylene + propylene" in product gas / (mass flow rate of product gas - mass flow rate of water in product gas - mass flow rate of methanol in product gas - mass flow rate of C4+ hydrocarbons in product gas) × 100%

Compared with the prior art, the present application has the following beneficial effects:
(1) The present application discloses a high-density fast fluidized bed reactor, which mainly comprises a reaction zone, a catalyst residence zone, and a gas-solid separation zone. The catalyst circulation intensity flowing from the catalyst residence zone to the reaction zone is as high as 500-1000 kg/(m²·s), thereby increasing the bed density of the reaction zone and achieving high bed density under high superficial gas velocity conditions, overcoming the negative correlation between the selectivity of low-carbon olefins and the feedstock conversion rate.
(2) In the method of the present application, the reaction contact time of the product gas in the high-density fast fluidized bed reactor is < 2 s, which effectively improves the carbon-based selectivity of "ethylene + propylene".
(3) In the method of the present application, the reaction of preparing low-carbon olefins from methanol is exothermic, providing heat for the catalytic cracking reaction of C₄+ hydrocarbons to prepare low-carbon olefins, achieving self-heat balance.
(4) In the method of the present application, continuous preparation of low-carbon olefins by coupling methanol and mixed hydrocarbons is achieved through a fluidized bed reaction-regeneration process.

### BRIEF DESCRIPTION OF THE DRAWINGS

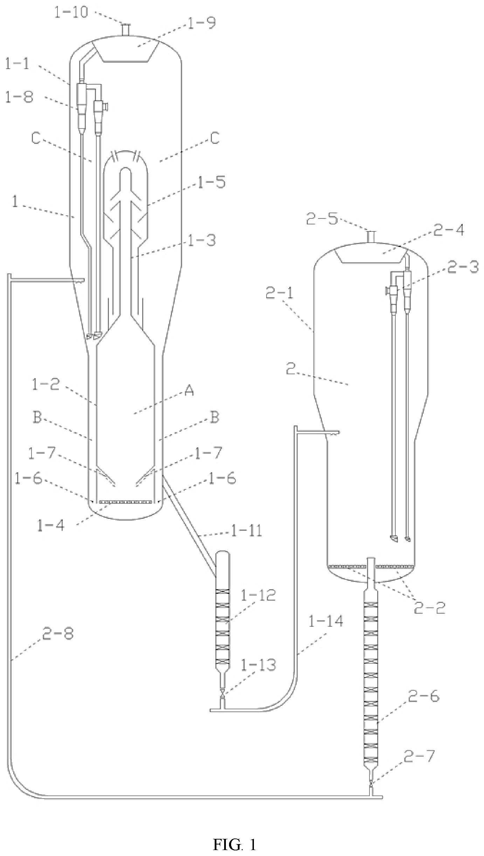

FIG. 1 is a schematic diagram of an apparatus according to one embodiment of the present application.

The reference numerals in FIG. 1 are explained as follows:
1 - High-density fast fluidized bed reactor;
1-1 Reactor outer shell, 1-2 Reactor inner shell, 1-3 Transport pipe, 1-4 Feedstock distributor, 1-5 First gas-solid separation device, 1-6 Fluidizing steam distributor, 1-7 Catalyst distribution pipe, 1-8 Reactor heat exchanger, 1-9 Reactor gas collection chamber; 1-10 Product gas transport pipe; 1-11 Catalyst extraction pipe; 1-12 Reactor stripper; 1-13 Spent slide valve; 1-14 Spent agent transport pipe;
2 - Fluidized bed regenerator;
2-1 Regenerator shell, 2-2 Regenerator distributor, 2-3 Third gas-solid separation device, 2-4 Regenerator gas collection chamber, 2-5 Flue gas transport pipe, 2-6 Regenerator stripper, 2-7 Regenerated slide valve, 2-8 Regenerated agent transport pipe.

### DETAILED DESCRIPTION OF THE EMBODIMENTS

The present application is described in detail below in conjunction with embodiments, but the present application is not limited to these embodiments.

Unless otherwise specified, the feedstocks and catalysts in the embodiments of the present application are all purchased through commercial channels.

In one specific embodiment, the present application provides an apparatus according to one embodiment, the structural schematic diagram of which is shown in FIG. 1. The apparatus comprises a high-density fast fluidized bed reactor (1) and a fluidized bed regenerator (2).
a. The high-density fast fluidized bed reactor (1) comprises: a reactor outer shell (1-1), a reactor inner shell (1-2), a transport pipe (1-3), a feedstock distributor (1-4), a first gas-solid separation device (1-5), a fluidizing steam distributor (1-6), a catalyst distribution pipe (1-7), a second gas-solid separation device (1-8), a reactor gas collection chamber (1-9), a product gas transport pipe (1-10), a catalyst extraction pipe (1-11), a reactor stripper (1-12), a spent slide valve (1-13), and a spent agent transport pipe (1-14). The region enclosed by the reactor inner shell (1-2) is the reaction zone (A). The annular region enclosed by the reactor outer shell (1-1) and the reactor inner shell (1-2) is the catalyst residence zone (B). The bottom of the reaction zone (A) and the bottom of the catalyst residence zone (B) are in communication. The region enclosed by the reactor outer shell (1-1) and the transport pipe (1-3) is the gas-solid separation zone (C). The catalyst residence zone (B) and the gas-solid separation zone (C) are in communication and located below the gas-solid separation zone (C). The feedstock distributor (1-4) is located at the bottom of the reaction zone (A). The transport pipe (1-3) is located in the central region of the middle and upper part of the high-density fast fluidized bed reactor. The bottom end of the transport pipe (1-3) is connected to the top of the reaction zone (A). The outlet of the transport pipe (1-3) is connected to the inlet of the first gas-solid separation device (1-5). The first gas-solid separation device (1-5) is located in the gas-solid separation zone (C). The catalyst outlet of the first gas-solid separation device (1-5) is located in the lower part of the gas-solid separation zone (C). The gas outlet of the first gas-solid separation device (1-5) is located in the upper part of the gas-solid separation zone (C). The fluidizing steam distributor (1-6) is located at the bottom of the catalyst residence zone (B). The catalyst distribution pipe (1-7) passes through the reactor inner shell (1-2) to connect the catalyst residence zone (B) and the reaction zone (A). The lower surface of the catalyst distribution pipe (1-7) is provided with openings. The second gas-solid separation device (1-8) is located in the gas-solid separation zone (C). The inlet of the second gas-solid separation device (1-8) is located in the gas-solid separation zone (C). The gas outlet of the second gas-solid separation device (1-8) is connected to the reactor gas collection chamber (1-9). The catalyst outlet of the second gas-solid separation device (1-8) is located in the lower part of the gas-solid separation zone (C). The reactor gas collection chamber (1-9) is located at the top of the high-density fast fluidized bed reactor. The product gas transport pipe (1-10) is connected to the top of the reactor gas collection chamber (1-9). The catalyst extraction pipe (1-11) passes through the reactor outer shell (1-1) and is located in the lower part of the catalyst residence zone (B). The reactor stripper (1-12) is connected to the catalyst extraction pipe (1-11). The inlet of the spent slide valve (1-13) is connected to the bottom of the reactor stripper (1-12). The outlet of the spent slide valve (1-13) is connected to the inlet of the spent agent transport pipe (1-14). The outlet of the spent agent transport pipe (1-14) is connected to the fluidized bed regenerator (2).
b. The fluidized bed regenerator (2) comprises: a regenerator shell (2-1), a regenerator distributor (2-2), a third gas-solid separation device (2-3), a regenerator gas collection chamber (2-4), a flue gas transport pipe (2-5), a regenerator stripper (2-6), a regenerated slide valve (2-7), and a regenerated agent transport pipe (2-8). The regenerator distributor (2-2) is located at the bottom of the fluidized bed regenerator (2). The third gas-solid separation device (2-3) is located in the upper part of the fluidized bed regenerator (2). The inlet of the third gas-solid separation device (2-3) is located in the upper part of the fluidized bed regenerator (2). The gas outlet of the third gas-solid separation device (2-3) is connected to the regenerator gas collection chamber (2-4). The catalyst outlet of the third gas-solid separation device (2-3) is located in the lower part of the fluidized bed regenerator (2). The regenerator gas collection chamber (2-4) is located at the top of the fluidized bed regenerator (2). The flue gas transport pipe (2-5) is connected to the top of the regenerator gas collection chamber (2-4). The regenerator stripper (2-6) is located outside the regenerator shell (2-1). The inlet pipe of the regenerator stripper (2-6) penetrates the regenerator shell (2-1) and opens above the regenerator distributor (2-2). The inlet of the regenerated slide valve (2-7) is connected to the bottom of the regenerator stripper (2-6). The outlet of the regenerated slide valve (2-7) is connected to the inlet of the regenerated agent transport pipe (2-8). The outlet of the regenerated agent transport pipe (2-8) is connected to the lower part of the gas-solid separation zone (C) of the high-density fast fluidized bed reactor (1).

In a preferred embodiment, the first gas-solid separation device (1-5) employs an inertial separator to achieve rapid separation of product gas and catalyst.

In a preferred embodiment, the second gas-solid separation device (1-8) employs one or more groups of gas-solid cyclone separators, each group of gas-solid cyclone separators comprising a first-stage gas-solid cyclone separator and a second-stage gas-solid cyclone separator.

In a preferred embodiment, the third gas-solid separation device (2-3) employs one or more groups of gas-solid cyclone separators, each group of gas-solid cyclone separators comprising a first-stage gas-solid cyclone separator and a second-stage gas-solid cyclone separator.

In one specific embodiment, a method for preparing low-carbon olefins by coupling methanol with mixed hydrocarbons according to the present application comprises the following steps:
a. The catalyst from the regenerated agent transport pipe (2-8) enters the gas-solid separation zone (C) of the high-density fast fluidized bed reactor (1), and then enters the catalyst residence zone (B). The gasified feedstock enters the reaction zone (A) from the feedstock distributor (1-4), contacts with the catalyst, and generates product gas containing low-carbon olefins. The product gas carries the catalyst through the transport pipe (1-3) into the first gas-solid separation device (1-5). After gas-solid separation, the catalyst enters the catalyst residence zone (B). Steam enters the catalyst residence zone (B) from the fluidizing steam distributor (1-6), and the steam carries a small amount of catalyst from the catalyst residence zone (B) into the gas-solid separation zone (C). The product gas and steam carrying the catalyst in the gas-solid separation zone (C) enter the second gas-solid separation device (1-8). After gas-solid separation, the gas enters the reactor gas collection chamber (1-9), and the catalyst returns to the catalyst residence zone (B). The product gas and steam enter the downstream section through the product gas transport pipe (1-10). The catalyst in the catalyst residence zone (B) enters the reaction zone (A) through the catalyst distribution pipe (1-7). The catalyst in the catalyst residence zone (B) enters the bottom of the reaction zone (A) through the bottom of the catalyst residence zone (B). The catalyst in the catalyst residence zone (B) enters the reactor stripper (1-12) through the catalyst extraction pipe (1-11). After stripping, the catalyst then enters the middle part of the fluidized bed regenerator (2) through the spent slide valve (1-13) and the spent agent transport pipe (1-14).
b. The regeneration gas enters the bottom of the fluidized bed regenerator (2) from the regenerator distributor (2-2). In the fluidized bed regenerator (2), the regeneration gas contacts with the catalyst, and the carbon deposits on the catalyst are burned and eliminated. The flue gas formed by combustion carries the catalyst into the third gas-solid separation device (2-3). After gas-solid separation, the flue gas enters the regenerator gas collection chamber (2-4) and then enters the downstream flue gas treatment system through the flue gas transport pipe (2-5). The catalyst returns to the bottom of the fluidized bed regenerator (2). The catalyst in the fluidized bed regenerator (2) enters the regenerator stripper (2-6). After stripping, it then enters the high-density fast fluidized bed reactor (1) through the regenerated slide valve (2-7) and the regenerated agent transport pipe (2-8).

### Example 1

Example 1 employs the apparatus shown in FIG. 1.

In Example 1, the feedstock is methanol and C₄-C₆ hydrocarbons. The methanol feed rate / C₄-C₆ hydrocarbon feed rate is approximately 1. The olefin content in the C₄-C₆ hydrocarbons is 91% wt, and the alkane content is 9% wt. The catalyst is a ZSM-5 molecular sieve catalyst.

The process operating conditions of the reaction zone (A) of the high-density fast fluidized bed reactor (1) are: superficial gas velocity of 1.5 m/s, temperature of 500°C, pressure of 250 kPa, bed density of 500 kg/m³, and reaction contact time of 2 s.

The process operating conditions of the catalyst residence zone (B) are: superficial gas velocity of 0.10 m/s, temperature of 500°C, and bed density of 670 kg/m³. The catalyst circulation intensity flowing from the catalyst residence zone (B) to the reaction zone (A) is 500 kg/(m²·s).

The regeneration gas is air. The process operating conditions of the fluidized bed regenerator (2) are: superficial gas velocity of 0.5 m/s, regeneration temperature of 600°C, regeneration pressure of 250 kPa, and bed density of 800 kg/m³.

The single-pass conversion rate of methanol is 99% wt, the single-pass conversion rate of mixed hydrocarbons is 79% wt, the carbon-based selectivity of "ethylene + propylene" is 87% wt, and the propylene/ethylene (mass ratio) is 3.1.

### Example 2

Example 2 employs the apparatus shown in FIG. 1.

In Example 2, the feedstock is methanol and C₄-C₆ hydrocarbons. The methanol feed rate / C₄-C₆ hydrocarbon feed rate is approximately 1. The olefin content in the C₄-C₆ hydrocarbons is 46% wt, and the alkane content is 54% wt. The catalyst is a ZSM-5 molecular sieve catalyst.

The process operating conditions of the reaction zone (A) of the high-density fast fluidized bed reactor (1) are: superficial gas velocity of 2.6 m/s, temperature of 600°C, pressure of 170 kPa, bed density of 310 kg/m³, and reaction contact time of 1.2 s.

The process operating conditions of the catalyst residence zone (B) are: superficial gas velocity of 0.2 m/s, temperature of 600°C, and bed density of 600 kg/m³. The catalyst circulation intensity flowing from the catalyst residence zone (B) to the reaction zone (A) is 720 kg/(m²·s).

The regeneration gas is air. The process operating conditions of the fluidized bed regenerator (2) are: superficial gas velocity of 2.0 m/s, regeneration temperature of 650°C, regeneration pressure of 170 kPa, and bed density of 200 kg/m³.

The single-pass conversion rate of methanol is 99.8% wt, the single-pass conversion rate of mixed hydrocarbons is 50% wt, the carbon-based selectivity of "ethylene + propylene" is 78% wt, and the propylene/ethylene (mass ratio) is 4.4.

### Example 3

Example 3 employs the apparatus shown in FIG. 1.

In Example 3, the feedstock is methanol and C₅-C₁₂ hydrocarbons. The methanol feed rate / C₅-C₁₂ hydrocarbon feed rate is approximately 1. The olefin content in the C₅-C₁₂ hydrocarbons is 64% wt, and the alkane content is 36% wt. The catalyst is a ZSM-5 molecular sieve catalyst.

The process operating conditions of the reaction zone (A) of the high-density fast fluidized bed reactor (1) are: superficial gas velocity of 4.1 m/s, temperature of 650°C, pressure of 110 kPa, bed density of 190 kg/m³, and reaction contact time of 0.8 s.

The process operating conditions of the catalyst residence zone (B) are: superficial gas velocity of 0.08 m/s, temperature of 650°C, and bed density of 720 kg/m³. The catalyst circulation intensity flowing from the catalyst residence zone (B) to the reaction zone (A) is 810 kg/(m²·s).

The regeneration gas is air. The process operating conditions of the fluidized bed regenerator (2) are: superficial gas velocity of 1.6 m/s, regeneration temperature of 690°C, regeneration pressure of 110 kPa, and bed density of 340 kg/m³.

The single-pass conversion rate of methanol is 99.9% wt, the single-pass conversion rate of mixed hydrocarbons is 77% wt, the carbon-based selectivity of "ethylene + propylene" is 81% wt, and the propylene/ethylene (mass ratio) is 7.8.

### Example 4

Example 4 employs the apparatus shown in FIG. 1.

In Example 4, the feedstock is methanol and C₅-C₁₂ hydrocarbons. The methanol feed rate / C₅-C₁₂ hydrocarbon feed rate is approximately 1. The olefin content in the C₅-C₁₂ hydrocarbons is 8% wt, and the alkane content is 92% wt. The catalyst is a ZSM-5 molecular sieve catalyst.

The process operating conditions of the reaction zone (A) of the high-density fast fluidized bed reactor (1) are: superficial gas velocity of 7.0 m/s, temperature of 680°C, pressure of 50 kPa, bed density of 150 kg/m³, and reaction contact time of 0.5 s.

The process operating conditions of the catalyst residence zone (B) are: superficial gas velocity of 0.02 m/s, temperature of 680°C, and bed density of 800 kg/m³. The catalyst circulation intensity flowing from the catalyst residence zone (B) to the reaction zone (A) is 1000 kg/(m²·s).

The regeneration gas is air. The process operating conditions of the fluidized bed regenerator (2) are: superficial gas velocity of 0.9 m/s, regeneration temperature of 750°C, regeneration pressure of 50 kPa, and bed density of 520 kg/m³.

The single-pass conversion rate of methanol is 99.9% wt, the single-pass conversion rate of mixed hydrocarbons is 65% wt, the carbon-based selectivity of "ethylene + propylene" is 75% wt, and the propylene/ethylene (mass ratio) is 9.3.

The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A method for preparing low-carbon olefins by coupling methanol with mixed hydrocarbons, comprising the steps of:
introducing gasified feedstock comprising methanol and mixed hydrocarbons into a reaction zone, contacting with a catalyst, reacting to generate stream I containing product gas of low-carbon olefins and the catalyst;
subjecting the stream I to gas-solid separation to obtain the product gas and the catalyst;
transporting the product gas to a downstream section; and
introducing a first part of the catalyst into the reaction zone, regenerating a second part of the catalyst, and introducing the regenerated catalyst into the reaction zone.

2. The method according to claim 1, wherein the mixed hydrocarbons in the feedstock are C₄+ hydrocarbons, comprising alkanes and olefins with carbon atom numbers ≥ 4.

3. The method according to claim 1 or 2, wherein the catalyst is selected from ZSM-5 molecular sieve catalyst.

4. The method according to any one of claims 1 to 3, wherein process operating conditions of the reaction zone comprise: superficial gas velocity of 1.5-7.0 m/s, temperature of 500-680°C, pressure of 50-250 kPa, bed density of 150-500 kg/m³, and reaction contact time of 0.5-2 s.

5. The method according to any one of claims 1 to 4, wherein the method is carried out in an apparatus for preparing low-carbon olefins from methanol and mixed hydrocarbons, the apparatus comprising a high-density fast fluidized bed reactor and a fluidized bed regenerator.

6. The method according to claim 5, wherein the high-density fast fluidized bed reactor comprises a reactor outer shell, a reactor inner shell, and a transport pipe;
wherein the reactor outer shell is provided with at least a feedstock inlet, a catalyst inlet, a gas phase product outlet, and a catalyst outlet;
wherein the reactor outer shell encloses an internal region;
wherein the reactor inner shell is located in a lower part of the internal region, and a region enclosed by the reactor inner shell is a reaction zone;
wherein the transport pipe is located in a middle and upper part of the internal region, and a bottom of the transport pipe is in communication with the reaction zone;
wherein a region enclosed by the reactor outer shell and the transport pipe is a gas-solid separation zone;
wherein the transport pipe is provided with an outlet, and the transport pipe is in communication with the gas-solid separation zone;
wherein an annular region enclosed by the reactor outer shell and the reactor inner shell is a catalyst residence zone;
wherein a bottom of the reaction zone and a bottom of the catalyst residence zone are in communication; and
wherein the catalyst residence zone and the gas-solid separation zone are in communication and located below the gas-solid separation zone.

7. The method according to claim 6, wherein process operating conditions of the catalyst residence zone comprise: superficial gas velocity of 0.02-0.2 m/s, temperature of 500-680°C, and bed density of 600-800 kg/m³.

8. The method according to claim 6 or 7, wherein a catalyst circulation intensity flowing from the catalyst residence zone to the reaction zone is 500-1000 kg/(m²·s).

9. The method according to any one of claims 6 to 8, wherein the high-density fast fluidized bed reactor comprises a catalyst distribution pipe, a fluidizing steam distributor, and a feedstock distributor;
wherein the catalyst distribution pipe passes through the reactor inner shell to connect the catalyst residence zone and the reaction zone;
wherein the fluidizing steam distributor is disposed at a bottom of the catalyst residence zone;
wherein the feedstock distributor is disposed at a bottom of the reaction zone; and
wherein a lower surface of the catalyst distribution pipe is provided with through holes.

10. The method according to any one of claims 6 to 9, wherein the high-density fast fluidized bed reactor comprises a first gas-solid separation device and a second gas-solid separation device;
wherein the first gas-solid separation device and the second gas-solid separation device are located in the gas-solid separation zone;
wherein an inlet of the first gas-solid separation device is in communication with the outlet of the transport pipe; and
wherein a catalyst outlet of the first gas-solid separation device is located in a lower part of the gas-solid separation zone, and a gas outlet of the first gas-solid separation device is located in an upper part of the gas-solid separation zone.

11. The method according to claim 10, wherein an inlet of the second gas-solid separation device is located in an upper part of the gas-solid separation zone; and
wherein a catalyst outlet of the second gas-solid separation device is located in a lower part of the gas-solid separation zone.

12. The method according to claim 10, wherein the first gas-solid separation device is an inertial separator.

13. The method according to claim 10, wherein the second gas-solid separation device comprises one or more groups of gas-solid cyclone separators, each group of gas-solid cyclone separators comprising a first-stage gas-solid cyclone separator and a second-stage gas-solid cyclone separator.

14. The method according to any one of claims 5 to 13, wherein the high-density fast fluidized bed reactor comprises a reactor gas collection chamber and a product gas transport pipe;
wherein the reactor gas collection chamber is located at a top of the high-density fast fluidized bed reactor;
wherein the product gas transport pipe is connected to a top of the reactor gas collection chamber; and
wherein a gas outlet of the second gas-solid separation device is in communication with the reactor gas collection chamber.

15. The method according to any one of claims 5 to 14, wherein the apparatus comprises a catalyst extraction pipe;
wherein the catalyst extraction pipe passes through the reactor outer shell and is located in a lower part of the catalyst residence zone, and the catalyst extraction pipe is in communication with a reactor stripper;
wherein an inlet of a spent slide valve is connected to a bottom of the reactor stripper, an outlet of the spent slide valve is connected to an inlet of a spent agent transport pipe, and an outlet of the spent agent transport pipe is in communication with the fluidized bed regenerator;
wherein a regenerator stripper is located at a bottom of the fluidized bed regenerator; and
wherein an inlet of a regenerated slide valve is connected to a bottom of the regenerator stripper, an outlet of the regenerated slide valve is connected to an inlet of a regenerated agent transport pipe, and an outlet of the regenerated agent transport pipe is connected to a lower part of the gas-solid separation zone of the high-density fast fluidized bed reactor.

16. The method according to any one of claims 5 to 15, wherein the fluidized bed regenerator comprises a regenerator shell, a regenerator distributor, a third gas-solid separation device, a regenerator gas collection chamber, and a flue gas transport pipe;
wherein the regenerator distributor is located at a bottom of the fluidized bed regenerator;
wherein the third gas-solid separation device is located in an upper part of the fluidized bed regenerator, an inlet of the third gas-solid separation device is located in an upper part of the fluidized bed regenerator, a gas outlet of the third gas-solid separation device is in communication with the regenerator gas collection chamber, and a catalyst outlet of the third gas-solid separation device is located in a lower part of the fluidized bed regenerator;
wherein the regenerator gas collection chamber is located at a top of the fluidized bed regenerator; and
wherein the flue gas transport pipe is connected to a top of the regenerator gas collection chamber.

17. The method according to claim 16, wherein an inlet pipe of the regenerator stripper penetrates the regenerator shell and opens above the regenerator distributor.

18. The method according to claim 16 or 17, wherein the third gas-solid separation device employs one or more groups of gas-solid cyclone separators, each group of gas-solid cyclone separators comprising a first-stage gas-solid cyclone separator and a second-stage gas-solid cyclone separator.

19. The method according to any one of claims 5 to 18, wherein process operating conditions of the fluidized bed regenerator are: superficial gas velocity of 0.5-2.0 m/s, regeneration temperature of 600-750°C, regeneration pressure of 50-250 kPa, and bed density of 200-800 kg/m³.

20. The method according to any one of claims 5 to 19, wherein a regeneration gas of the fluidized bed regenerator is air.

21. The method according to any one of claims 10 to 20, wherein the stream I enters the first gas-solid separation device through the transport pipe, and the catalyst after a first gas-solid separation enters the catalyst residence zone.

22. The method according to any one of claims 6 to 20, wherein steam enters the catalyst residence zone from the fluidizing steam distributor, and the steam carries a part of the catalyst in the catalyst residence zone into the gas-solid separation zone; and
wherein product gas and steam carrying a part of the catalyst in the gas-solid separation zone enter the second gas-solid separation device, and after a second gas-solid separation, gas enters the reactor gas collection chamber, and separated catalyst returns to the catalyst residence zone.

23. The method according to claim 22, wherein the product gas and steam after the second gas-solid separation enter a downstream section through the product gas transport pipe;
wherein a first part of the catalyst in the catalyst residence zone enters the reaction zone through the catalyst distribution pipe;
wherein a second part of the catalyst in the catalyst residence zone enters a bottom of the reaction zone through a bottom of the catalyst residence zone; and
wherein a third part of the catalyst in the catalyst residence zone enters the reactor stripper through the catalyst extraction pipe.
